# NEW EUROPEAN PATENT SPECIFICATION

(11) **EP 2 493 574 B3**
(45) Date of publication of this specification: **10.05.2017**
(45) Mention of the grant of the patent: 25.06.2014
(21) Application number: 10775769.2
(22) Date of filing: 27.10.2010
(51) Int. Cl.: A61Q 1/02, A61K 8/02, A61K 8/69, A61K 8/91, A61K 8/89, A61K 8/92, A61K 8/70

(54) **MAKEUP AND/OR CARE COMPOSITION CONTAINING PIGMENTS COATED WITH A FLUORO COMPOUND AND A VINYL POLYMER CONTAINING A CARBOSILOXANE DENDRIMER-BASED UNIT**
MAKEUP- UND/ODER PFLEGEZUSAMMENSETZUNG AUS MIT EINER FLUORVERBINDUNG BESCHICHTEN PIGMENTEN UND EINEM VINYLPOLYMER MIT EINER CARBOSILOXANEINHEIT AUF DENDRIMERBASIS
COMPOSITION DE MAQUILLAGE ET/OU DE SOIN CONTENANT DES PIGMENTS ENROBÉS D'UN COMPOSÉ FLUORÉ ET UN POLYMÈRE VINYLIQUE CONTENANT UNE UNITÉ À BASE DE DENDRIMÈRES DE CARBOSILOXANE

(30) Priority: 29.10.2009 FR 0957629; 05.11.2009 US 258248 P
(43) Date of publication of application: 05.09.2012
(73) Proprietor: L'Oréal, 75008 Paris (FR)
(72) Inventor: ARNAUD, Pascal, F-94240 L'hay Les Roses (FR); BEAUMARD, Sophie, F-94800 Villejuif (FR)
(74) Representative: Leonard, Armelle
(86) International application number: PCT/EP2010/066236
(87) International publication number: WO 2011/051323

(56) References cited:
- EP-A1- 1 815 840
- EP-A1- 1 862 162
- FR-A1- 2 872 032
- US-A- 3 597 250

## Description

The present invention relates to the field of caring for and making up keratin materials, and in particular the skin and mucous membranes. More particularly, the present invention relates to compositions for giving the skin a satin effect and which have improved remanence of this satin effect over time.

Cosmetic compositions, for instance foundations, are commonly used to give the skin, especially the face, a colour and an aesthetic effect. These makeup products generally contain oils, pigments, fillers and optionally additives such as cosmetic or dermatological active agents.

Consumers are increasingly in search of makeup products that do not produce a dull effect when they are applied to the skin. On the contrary, they are in search of a luminous makeup result that is neither too matt nor too glossy: this will be termed a satin effect in the rest of the description.

Excessive mattness is not aesthetic since it gives the skin an appearance of dryness and a powdery effect that does not bring out the features of the face. At the opposite end of the scale, excessive gloss is not desired either, since this result is associated with the presence of sebum or sweat on the skin.

Moreover, consumers desire remanence throughout the day of this satin makeup result, since an increase in gloss is frowned upon for the reasons described previously.

It is known to those skilled in the art to use polymers in order to obtain remanence of makeup throughout the day.

These polymers are of very different chemical nature and may be conveyed in the fatty phase or in the aqueous phase. Examples of these polymers that may be mentioned include silicone resins, polyacrylates, latices, etc.

Although these polymers do indeed provide remanence properties, they are usually accompanied by discomfort either during the application of the product (difficulty in spreading, tackiness, etc.) or throughout the day (tautness, mask effect, etc.).

There is thus still a need for cosmetic compositions that have a satin effect and improved remanence of this satin effect, which are pleasant and easy to apply, while at the same time maintaining satisfactory comfort on application i.e. not causing any sensation of tautness or mask effect throughout the day and/or not inducing any greasy or tacky sensation during their application.

The object of the present invention is to satisfy these needs.

The Applicant has found, unexpectedly, that the introduction into a makeup composition of particulate dyestuffs having a coating that is also specific (fluoro coating) in combination with a specific silicone polymer (vinyl polymer containing at least one carbosiloxane dendrimer-based unit) makes it possible to obtain a makeup result that is satiny when compared with other coatings of particulate dyestuffs, and that has very good remanence properties of this satin effect, compared with other polymers with a remanence effect.

The present invention thus relates to a cosmetic composition for making up and/or caring for keratin materials, in particular the skin, comprising a liquid fatty phase, at least one vinyl polymer containing at least one carbosiloxane dendrimer-based unit and at least one particulate dyestuff coated with at least one fluoro compound ; the said vinyl polymer having as INCI name **ACRYLATES/POLYTRIMETHYL SILOXYMETHACRYLATE COPOLYMER.**

Patent application EP 1 862 162 discloses a composition for making up or caring for keratin materials, in particular the skin, comprising a liquid fatty phase comprising at least one vinyl polymer containing at least one carbosiloxane dendrimer-based unit and at least one olefin copolymer, for obtaining a comfortable makeup, without a tacky sensation, which spreads easily. The said composition of that document may also comprise a pulverulent phase containing particulate dyestuffs (fillers, pigments and nacres) intended to give matt and coverage properties. The said particulate dyestuffs are optionally treated with a hydrophobic treating agent to make them compatible with the organic phase of the composition, such as silicones, fatty acids, metal soaps, fluoro compounds or amino acids, and mixtures thereof. Only pigments coated with amino acids are described in the formulation examples of that document.

To the Applicant's knowledge, it has never been specifically proposed to use at least one particulate dyestuff coated with at least one fluoro compound in combination selectively with a vinyl polymer containing at least one carbosiloxane dendrimer-based unit **having as INCI name ACRYLATES/POLYTRIMETHYL SILOXYMETHACRYLATE COPOLYMER**; this specific combination having shown unexpected results in terms of satin effect and remanence of this satin effect, relative to the teaching of the prior art (amino acid coating with a matt effect).

A subject of the present invention is also the use in a cosmetic composition for making up and/or caring for keratin materials, in particular the skin, of at least one vinyl polymer containing at least one carbosiloxane dendrimer-based unit having as INCI name ACRYLATES/POLYTRIMETHYL SILOXYMETHACRYLATE COPOLYMER, in combination with at least one particulate dyestuff coated with at least one fluoro compound, to give the said composition a satin effect and improved remanence of the said satin effect on the keratin materials, in particular the skin.

The invention also relates to the use in a cosmetic composition for making up and/or caring for keratin materials, in particular the skin, of at least one particulate dyestuff coated with at least one fluoro compound, for giving the said composition a satin effect on the keratin materials, in particular the skin.

According to the invention, the term "satin effect" especially means a gloss value measured using a polarimetric camera, after applying a composition according to the invention to the said keratin material, of less than 5, preferably less than or equal to 4 and better still less than or equal to 3.

The present invention also relates to a non-therapeutic process for making up and/or caring for keratin materials, in particular the skin, comprising at least the step of applying to the said keratin material at least one coat of a composition according to the invention.

### VINYL POLYMER GRAFTED WITH A CARBOSILOXANE DENDRIMER

The vinyl polymer used according to the invention especially has a backbone and at least one side chain, which comprises a carbosiloxane dendrimer-based unit having a carbosiloxane dendrimer structure.

Vinyl polymers comprising at least one carbosiloxane dendrimer unit as described in patent applications WO 03/045 337 and EP 963 751 by the company Dow Corning may be used in particular.

The term "carbosiloxane dendrimer structure" in the context of the present invention represents a structure with branched groups of high molecular masses with high regularity in the radial direction starting from the bond to the backbone. Such carbosiloxane dendrimer structures are described in the form of a highly branched siloxane-silylalkylene copolymer in the laid-open Japanese patent application Kokai 9-171 154.

According to one preferred embodiment, a vinyl polymer grafted with a carbosiloxane dendrimer may be the product of polymerization of:
(A) from 0 to 99.9 parts by weight of one or more acrylate or methacrylate monomers; and
(B) from 100 to 0.1 part by weight of an acrylate or methacrylate monomer of a tris[tri(trimethylsiloxy)-silylethyldimethylsiloxy]silylpropyl carbosiloxane dendrimer.

According to one embodiment, a vinyl polymer containing at least one carbosiloxane dendrimer-based unit may comprise a tris[tri(trimethylsiloxy)silylethyldimethylsiloxy]sil-ylpropyl carbosiloxane dendrimer-based unit corresponding to one of the formulae: 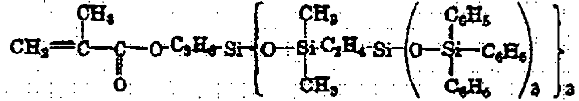 or

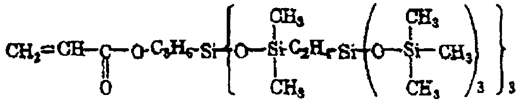

Such compounds have as INCI name ACRYLATES/POLYTRIMETHYL SILOXYMETHACRYLATE COPOLYMER.

According to one preferred mode, a vinyl polymer containing at least one carbosiloxane dendrimer-based unit used in the invention comprises at least one butyl acrylate monomer.

The carbosiloxane dendrimer may be manufactured according to the process for manufacturing a branched silalkylene siloxane described in Japanese patent application Hei 9-171 154.

For example, it may be produced by subjecting an organosilicon compound containing a hydrogen atom linked to a silicon atom and an organosilicon compound containing an alkenyl group, to a hydrosilylation reaction.

The organosilicon compound may be represented by 3-methacryloxypropyl-tris(dimethylsiloxy)silane, 3-acryloxy-propyltris(dimethylsiloxy)silane and 4-vinylphenyl-tris(dimethylsiloxy)silane. The organosilicon compound that contains an alkenyl group may be represented by vinyltris(trimethylsiloxy)silane, vinyltris(dimethylphenylsiloxy)silane, and 5-hexenyl-tris(trimethylsiloxy)silane.

The hydrosilylation reaction is performed in the presence of a chloroplatinic acid, a complex of vinylsiloxane and of platinum, or a similar transition metal catalyst.

A vinyl polymer containing at least one carbosiloxane dendrimer-based unit may be obtained by copolymerization of the components (A) and (B), or by polymerization of the component (B) alone.

The polymerization may be a free-radical polymerization or an ionic polymerization, but free-radical polymerization is preferred.

The polymerization may be performed by bringing about a reaction between the components (A) and (B) in a solution for a period of from 3 to 20 hours in the presence of a radical initiator at a temperature of from 50°C to 150°C.

A suitable solvent for this purpose is hexane, octane, decane, cyclohexane or a similar aliphatic hydrocarbon; benzene, toluene, xylene or a similar aromatic hydrocarbon; diethyl ether, dibutyl ether, tetrahydrofuran, dioxane or similar ethers; acetone, methyl ethyl ketone, methyl isobutyl ketone, diisobutyl ketone or similar ketones; methyl acetate, ethyl acetate, butyl acetate, isobutyl acetate or similar esters; methanol, ethanol, isopropanol, butanol or similar alcohols; octamethylcyclotetrasiloxane, decamethylcyclopentasiloxane, hexamethyldisiloxane, octamethyltrisiloxane or a similar organosiloxane oligomer.

A radical initiator may be any compound known in the art for standard free-radical polymerization reactions. Specific examples of such radical initiators are 2,2'-azobis(isobutyronitrile), 2,2'-azobis(2-methylbutyronitrile), 2,2'-azobis(2,4-dimethylvalero-nitrile) or similar compounds of azobis type; benzoyl peroxide, lauroyl peroxide, tert-butyl peroxybenzoate, tert-butyl peroxy-2-ethylhexanoate or a similar organic peroxide. These radical initiators may be used alone or in a combination of two or more. The radical initiators may be used in an amount of from 0.1 to 5 parts by weight per 100 parts by weight of the components (A) and (B). A chain-transfer agent may be added. The chain-transfer agent may be 2-mercaptoethanol, butyl mercaptan, n-dodecyl mercaptan, 3-mercaptopropyltrimethoxysilane, a polydimethylsiloxane containing a mercaptopropyl group or a similar compound of mercapto type; methylene chloride, chloroform, carbon tetrachloride, butyl bromide, 3-chloropropyltrimethoxysilane or a similar halogenated compound.

In the manufacture of the polymer of vinyl type, after the polymerization, the residual unreacted vinyl monomer may be removed under conditions of heating under vacuum.

To facilitate the preparation of the mixture of the starting material of cosmetic products, the number-average molecular mass of the vinyl polymer containing a carbosiloxane dendrimer may be chosen within the range between 3000 and 2 000 000 and preferably between 5000 and 800 000. It may be a liquid, a gum, a paste, a solid, a powder or any other form. The preferred forms are solutions consisting of the dilution of a dispersion or of a powder in solvents.

The vinyl polymer may be a dispersion of a polymer of vinyl type having a carbosiloxane dendrimer structure in its molecular side chain, in a liquid such as a silicone oil, an organic oil, an alcohol or water.

The silicone oil may be a dimethylpolysiloxane with the two molecular ends capped with trimethylsiloxy groups, a copolymer of methylphenylsiloxane and of dimethylsiloxane having the two molecular ends capped with trimethylsiloxy groups, a copolymer of methyl-3,3,3-trifluoropropylsiloxane and of dimethylsiloxane having the two molecular ends capped with trimethylsiloxy groups, or similar unreactive linear silicone oils, and also hexamethylcyclotrisiloxane, octamethylcyclotetrasiloxane, decamethylcyclopentasiloxane, dodecamethylcyclohexasiloxane or a similar cyclic compound. In addition to the unreactive silicone oils, modified polysiloxanes containing functional groups such as silanol groups, amino groups and polyether groups on the ends or within the molecular side chains may be used.

The organic oils may be isododecane, liquid paraffin, isoparaffin, hexyl laurate, isopropyl myristate, myristyl myristate, cetyl myristate, 2-octyldodecyl myristate; isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cocoa oil, jojoba oil, gum oil, sunflower oil, soybean oil, camellia oil, squalane, castor oil, mink oil, cottonseed oil, coconut oil, egg yolk oil, beef tallow, lard, polypropylene glycol monooleate, neopentyl glycol 2-ethylhexanoate or a similar glycol ester oil; triglyceryl isostearate, the triglyceride of a fatty acid of coconut oil, or a similar oil of a polyhydric alcohol ester; polyoxyethylene lauryl ether, polyoxypropylene cetyl ether or a similar polyoxyalkylene ether.

The alcohol may be any type that is suitable for use in combination with a cosmetic product starting material. For example, it may be methanol, ethanol, butanol, isopropanol or similar lower alcohols. A solution or a dispersion of the alcohol should have a viscosity within the range from 10 to 10⁹ mPa at 25°C. To improve the sensory use properties in a cosmetic product, the viscosity should be within the range from 100 to 5 x 10⁸ mPa.s.

The solutions and dispersions may be readily prepared by mixing the vinyl polymer having a carbosiloxane dendrimer structure with a silicone oil, an organic oil, an alcohol or water. The liquids may be present in the step of polymerization of the polymer of vinyl type having a carbosiloxane dendrimer structure. In this case, the unreacted residual vinyl monomer should be completely removed by heat treatment of the solution or dispersion under atmospheric pressure or reduced pressure.

In the case of a dispersion, the dispersity of the polymer of vinyl type may be improved by adding a surfactant.

Such an agent may be hexylbenzenesulfonic acid, octylbenzenesulfonic acid, decylbenzenesulfonic acid, dodecylbenzenesulfonic acid, cetylbenzenesulfonic acid, myristylbenzenesulfonic acid or anionic surfactants of the sodium salts of these acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow-trimethylammonium hydroxide, coconut oil-trimethylammonium hydroxide, or a similar cationic surfactant; a polyoxyalkylene alkyl ether, a polyoxyalkylenealkylphenol, a polyoxyalkylene alkyl ester, the sorbitol ester of polyoxyalkylene, polyethylene glycol, polypropylene glycol, an ethylene oxide additive of diethylene glycol trimethylnonanol, and nonionic surfactants of polyester type, and also mixtures.

In addition, the solvents and dispersions may be combined with iron oxide suitable for use with cosmetic products, or a similar pigment, and also zinc oxide, titanium oxide, silicon oxide, mica, talc or similar mineral oxides in powder form. In the dispersion, a mean particle diameter of the polymer of vinyl type may be within a range of between 0.001 and 100 microns and preferably between 0.01 and 50 microns. The reason for this is that, outside the recommended range, a cosmetic product mixed with the emulsion will not have a nice enough feel on the skin or to the touch, or sufficient spreading properties or a pleasant feel.

A vinyl polymer contained in the dispersion or the solution may have a concentration in the range between 0.1% and 95% by weight and preferably between 5% and 85% by weight. However, to facilitate the handling and the preparation of the mixture, the range should preferably be between 10% and 75% by weight.

According to one preferred embodiment, a vinyl polymer grafted in the sense of the present invention may be conveyed in an oil or a mixture of oils, which are preferably volatile, chosen in particular from silicone oils and hydrocarbon-based oils, and mixtures thereof.

According to one particular embodiment, a silicone oil that is suitable for use in the invention may be cyclopentasiloxane.

According to another particular embodiment, a hydrocarbon-based oil that is suitable for use in the invention may be isododecane.

In a preferred embodiment, a vinyl polymer grafted in the sense of the present invention is conveyed in isododécane.

Vinyl polymers grafted with at least one carbosiloxane dendrimer-based unit that may be particularly suitable for use in the present invention are the polymers sold under the names TIB 4-100, TIB 4-101, TIB 4-120, TIB 4-130, TIB 4-200, FA 4002 ID (TIB 4-202), TIB 4-220 and FA 4001 CM (TIB 4-230) by the company Dow Corning. The polymers sold under the names FA 4002 ID (TIB 4-202) and FA 4001 CM (TIB 4-230) by the company Dow Corning will preferably be used (INCI name ACRYLATES/POLYTRIMETHYLSILOXYMETHACRYLATE COPOLYMER).

According to one particular mode of the invention, the said vinyl polymer containing at least one carbosiloxane dendrimer-based unit is present in the composition in a content of greater than or equal to 1% by weight of active material relative to the total weight of the said composition.

In particular, the said vinyl polymer containing at least one carbosiloxane dendrimer-based unit is present in an active material content ranging from 1% to 20% by weight relative to the total weight of the composition, in particular ranging from 2% to 15% by weight and more particularly ranging from 3% to 10% by weight of active material of vinyl polymer containing at least one carbosiloxane dendrimer-based unit relative to the total weight of the said composition.

### PARTICULATE DYESTUFFS TREATED WITH A FLUORO COMPOUND

The compositions of the invention comprise at least one particulate dyestuff coated with at least one fluoro compound.

The terms "coated" and "surface-treated" will be used without distinction in the rest of the description.

The term "fluoro compound" means a compound comprising at least one fluorine atom.

### Particulate dyestuffs

The particulate dyestuff coated with at least one fluoro compound may be chosen from pigments and nacres, and mixtures thereof.

The term "pigments" should be understood as meaning white or coloured, mineral or organic particles of any form, which are insoluble in the physiological medium, and which are intended to colour the composition.

The term "nacres" should be understood as meaning iridescent particles of any form, produced especially by certain molluscs in their shell, or else synthesized.

The pigments may be white or coloured, and mineral and/or organic. Among the mineral pigments that may be mentioned are titanium dioxide, zirconium oxide or cerium oxide, and also zinc oxide, iron oxide (black, yellow or red) or chromium oxide, manganese violet, ultramarine blue, chromium hydrate and ferric blue, and metal powders, for instance aluminium powder or copper powder.

Among the organic pigments that may be mentioned are carbon black, pigments of D & C type, and lakes based on cochineal carmine or on barium, strontium, calcium or aluminium.

Mention may also be made of pigments with an effect, such as particles comprising a natural or synthetic, organic or mineral substrate, for example glass, acrylic resins, polyester, polyurethane, polyethylene terephthalate, ceramics or aluminas, the said substrate being uncoated or coated with metallic substances, for instance aluminium, gold, silver, platinum, copper or bronze, or with metal oxides, for instance titanium dioxide, iron oxide or chromium oxide, and mixtures thereof.

The nacreous pigments may be chosen from white nacreous pigments such as mica coated with titanium or with bismuth oxychloride, coloured nacreous pigments such as titanium mica coated with iron oxides, titanium mica coated especially with ferric blue or with chromium oxide, titanium mica coated with an organic pigment of the abovementioned type, and also nacreous pigments based on bismuth oxychloride. Interference pigments, especially liquid-crystal or multilayer interference pigments, may also be used.

As illustrations of nacres that may be introduced as interference pigments into the first composition, mention may be made especially of the gold-coloured nacres sold especially by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold especially by the company Merck under the name Bronze fine (17384) (Colorona) and Bronze (17353) (Colorona) and by the company Engelhard under the name Super bronze (Cloisonne); the orange nacres sold especially by the company Engelhard under the name Orange 363C (Cloisonne) and Orange MCR 101 (Cosmica) and by the company Merck under the name Passion orange (Colorona) and Matte orange (17449) (Microna); the brown nacres sold especially by the company Engelhard under the name Nu-antique copper 340XB (Cloisonne) and Brown CL4509 (Chromalite); the nacres with a copper tint sold especially by the company Engelhard under the name Copper 340A (Timica); the nacres with a red tint sold especially by the company Merck under the name Sienna fine (17386) (Colorona); the nacres with a yellow tint sold especially by the company Engelhard under the name Yellow (4502) (Chromalite); the red nacres with a gold tint sold especially by the company Engelhard under the name Sunstone G012 (Gemtone); the pink nacres sold especially by the company Engelhard under the name Tan opale G005 (Gemtone); the black nacres with a gold tint sold especially by the company Engelhard under the name Nu antique bronze 240 AB (Timica), the blue nacres sold especially by the company Merck under the name Matte blue (17433) (Microna), the white nacres with a silvery tint sold especially by the company Merck under the name Xirona Silver, and the golden-green pink-orange nacres sold especially by the company Merck under the name Indian summer (Xirona), and mixtures thereof.

In a particular embodiment, the particulate dyestuffs are chosen from mineral pigments, preferably titanium dioxide, iron oxides and mixtures thereof.

In a preferred embodiment, the particulate dyestuffs are iron oxides.

### Fluoro compound of the coating

The abovementioned particulate dyestuffs are totally or partially surface-treated with at least one fluoro compound.

In particular, the fluoro compound is chosen from perfluoroalkanes, perfluoroalkyl phosphates, perfluoroalkylsilanes, perfluoroalkylsilazanes, polyhexafluoropropylene oxides, perfluoropolyethers, polytetrafluoropolyethylene (PTFE), polyorganosiloxanes comprising perfluoroalkyl groups, such as perfluoroalkyl dimethicones and perfluoroalkyl trialkoxysilanes, and mixtures thereof.

According to one particular mode, the composition according to the invention comprises a particulate dyestuff coated with at least one fluoro compound chosen from iron oxides coated with perfluoroalkyl phosphate or perfluoropolymethyl isopropyl ether, and titanium dioxide coated with perfluoroalkyl phosphate, and mixtures thereof.

Perfluoropolyethers are especially described in patent application EP-A-486 135, and sold under the trade name Fomblin by the company Montefluos.

Perfluoroalkyl phosphates are described in particular in patent application JP-H05-86984. The perfluoroalkyl phosphate diethanolamines sold by Asahi Glass under the reference AsahiGuard AG530 may be used.

The perfluoroalkanes may be linear or cyclic perfluoroalkanes. Among the linear perfluoroalkanes, mention may be made of the series of linear alkanes, such as perfluorooctane, perfluorononane or perfluorodecane. Among the cyclic perfluoroalkanes, mention may be made of perfluorocycloalkanes, perfluoro(alkylcycloalkanes), perfluoropolycycloalkanes and aromatic perfluorohydrocarbons (perfluoroarenes). Among the perfluoroalkanes, mention may also be made of hydrocarbon-based organoperfluoro compounds comprising at least one heteroatom.

Among the perfluorocycloalkanes and perfluoro-(alkylcycloalkanes), mention may be made of perfluorodecalin sold under the name Flutec PP5 GMP by the company Rhodia, perfluoro(methyldecalin) and perfluoro(C3-C5 alkylcyclohexanes) such as perfluoro-(butylcyclohexane).

Among the perfluoropolycycloalkanes, mention may be made of bicyclo[3.3.1]nonane derivatives such as perfluorotrimethylbicyclo[3.3.1]nonane, adamantane derivatives such as perfluorodimethyladamantane, and hydrogenated perfluorophenanthrene derivatives such as tetracosafluorotetradecahydrophenanthrene.

Among the perfluoroarenes, mention may be made of perfluoronaphthalene derivatives, for instance perfluoronaphthalene and perfluoromethyl-1-naphthalene.

As examples of commercial references of pigments surface-treated with at least one fluoro compound, mention may be made of:
- yellow iron oxide coated with perfluoroalkyl phosphate, sold under the reference PFX 5 Sunpuro Yellow C33-9001 by the company Daito Kasei (INCI name iron oxides (and) C9-15 FLUOROALCOHOL PHOSPHATES),,
- red iron oxide coated with perfluoroalkyl phosphate, sold under the reference PFX 5 Sunpuro Red C33-8001 by the company Daito Kasei (INCI name iron oxides (and) C9-15 FLUOROALCOHOL PHOSPHATES),
- black iron oxide coated with perfluoroalkyl phosphate, sold under the reference PFX 5 Sunpuro Black C33-7001 by the company Daito Kasei (INCI name iron oxides (and) C9-15 FLUOROALCOHOL PHOSPHATES),,
- titanium dioxide coated with perfluoroalkyl phosphate, sold under the reference PF 5 TiO2 A 100 by the company Daito Kasei (INCI name titanium dioxide (and) C9-15 FLUOROALCOHOL PHOSPHATES),,
- titanium dioxide coated with perfluoroalkyl phosphate, sold under the reference PFX 5 TiO2 CR 50 by the company Daito Kasei (INCI name titanium dioxide (and) C9-15 FLUOROALCOHOL PHOSPHATES),,
- yellow iron oxide coated with perfluoropolymethyl isopropyl ether, sold under the reference Iron oxide yellow BF-25-3 by the company Toshiki,
- DC Red 7 coated with perfluoropolymethyl isopropyl ether, sold under the reference D&C Red 7 FHC by the company Cardre Inc.,
- DC Red 6 coated with PTFE, sold under the reference T 9506 by the company Warner-Jenkinson.

In a particular embodiment, the fluoro compound of the coating is chosen from perfluoroalkyl phosphate, in particular C₉-C₁₅ fluoroalcohol phosphate.

In a preferred embodiment, the particulate dyestuff coated with at least one fluoro compound are chosen from titanium dioxide coated with perfluoroalkyl phosphate preferably titanium dioxide coated with C₉-C₁₅ fluoroalcohol phosphate, iron oxides coated with perfluoroalkyl phosphate preferably iron oxide coated with C₉-C₁₅ fluoroalcohol phosphate, and mixtures thereof.

In a particular embodiment, the cosmetic composition according to the invention comprises a liquid fatty phase, at least one vinyl polymer containing at least one carbosiloxane dendrimer-based unit and at least titanium dioxide and/or iron oxides coated with C₉-C₁₅ fluoroalcohol phosphates.

### Coating

The surface-treated particulate dyestuffs may be prepared according to surface-treatment techniques of chemical, electronic, mechanochemical or mechanical nature that are well known to those skilled in the art. Commercial products may also be used.

The surface agent may be absorbed or adsorbed onto the particulate dyestuffs by evaporation of solvent, chemical reaction and creation of a covalent bond.

According to one variant, the surface treatment consists of coating of the particulate dyestuffs.

The coating may represent from 1% to 300% by weight relative to the weight of the untreated particulate dyestuffs, for example from 5% to 200% and in particular from 10% to 100% by weight relative to the weight of the untreated particulate dyestuffs.

The coating may represent from 0.1% to 10% by weight and in particular from 1% to 5% by weight relative to the total weight of the coated particulate dyestuff.

The coating may be produced, for example, by adsorption of a liquid surface agent onto the surface of the pulverulent dyestuffs by simple mixing with stirring of the particulate dyestuffs and of the said surface agent, optionally at elevated temperature, prior to the incorporation of the particles into the other ingredients of the makeup or care composition.

The coating may be produced, for example, by chemical reaction of a surface agent with the surface of the particulate dyestuffs and creation of a covalent bond between the surface agent and the pulverulent dyestuffs. This method is especially described in patent US 4 578 266.

The chemical surface treatment may consist in diluting the surface agent in a volatile solvent, dispersing the particulate dyestuffs in this mixture, and then slowly evaporating off the volatile solvent, so that the surface agent becomes deposited on the surface of the particulate dyestuffs.

In particular, the said particulate dyestuff coated with at least one fluoro compound is present in a content ranging from 0.5% to 40% by weight relative to the total weight of the said composition, preferably from 1% to 30% by weight relative to the total weight of the said composition and even more preferentially from 5% to 15% by weight relative to the total weight of the said composition.

According to one particular mode, the composition according to the invention comprising at least one vinyl polymer containing at least one carbosiloxane dendrimer-based unit and at least one particulate dyestuff coated with at least one fluoro compound increases the gloss of the keratin material onto which the composition according to the invention is applied, measured according to the protocol described below, it being understood that the gloss value measured after application is less than 5, preferably less than or equal to 4 and better still less than or equal to 3 (corresponding to the desired satin effect).

The protocol for measuring the desired satin effect and the remanence of this effect imparted by the application of a composition according to the invention onto a keratin material, in particular the skin, consists in:
- measuring the gloss of skin freed of makeup at T0 using a polarimetric camera, by analysis of the image resulting from the subtraction (P-C) of the images in parallel (P) and crossed (C) polarized light and measurement of the average greyscale of the brightest 5% of pixels corresponding to the gloss areas;
- applying with the bare fingers 100 mg of test composition onto the said skin freed of makeup and measuring, after a drying time of 15 minutes, the gloss of the made-up cheek (Timm);
- taking a second measurement after 3 hours in an air-conditioned room of the same made-up cheek (T3h);
- calculating the difference (Timm - T0) to measure the effect of the makeup on the skin;
- calculating the difference (T3h - Timm) to measure the remanence of the makeup effect on the skin.

A positive gloss value for (Timm-T0) but less than 5, preferably less than or equal to 4 and better still less than or equal to 3, characterizes the satin effect imparted onto the skin by the said composition of the invention.

According to one particular mode of the invention, the weight ratio between the said vinyl polymer containing at least one carbosiloxane dendrimer-based unit (as active material) and the said particulate dyestuff coated with at least one fluoro compound ranges from 0.1 to 1. In particular, the weight ratio between the said vinyl polymer containing at least one carbosiloxane dendrimer-based unit (as active material) and the said particulate dyestuff coated with at least one fluoro compound ranges from 0.2 to 0.7 and preferably from 0.25 to 0.5.

Besides the particulate dyestuffs coated with at least one fluoro compound used according to the invention, the composition according to the invention may also comprise at least one filler, of organic or mineral nature, which can especially give it additional texture or coverage properties. The filler content used in the composition of the invention will be adapted so as not to adversely affect the satin effect and the remanence of this satin effect that are desired in the context of the invention and provided by the combination of particulate dyestuffs coated with at least one fluoro compound and of the vinyl polymer containing at least one carbosiloxane dendrimer-based unit.

The term "filler" should be understood as meaning colourless or white, solid particles of any form, which are in a form that is insoluble and dispersed in the medium of the composition. These particles, of mineral or organic nature, can give body or rigidity to the composition and/or softness and uniformity to the makeup.

The fillers may be of lamellar, globular, spherical or fibrous form or of any other form intermediate between these defined forms.

The fillers may or may not be surface coated, and in particular they may be surface-treated with silicones, amino acids, fluoro derivatives or any other substance that promotes the dispersion and compatibility of the filler in the composition. Among the fillers that may be used in the compositions according to the invention, mention may be made of talc, mica, kaolin, bentone, precipitated calcium carbonate, magnesium carbonate, magnesium hydrogen carbonate, hydroxyapatite, boron nitride, glass or ceramic microcapsules, composites of silica and titanium dioxide, such as the TSG series sold by Nippon Sheet Glass, polyamide powder (Nylon^{®} Orgasol from Atochem), poly-β-alanine powder, polyethylene powder, polytetrafluoroethylene (Teflon^{®}) powders, lauroyllysine, starch, hollow polymer microspheres, such as Expancel (Nobel Industrie), metal soaps derived from organic carboxylic acids containing from 8 to 22 carbon atoms and preferably from 12 to 18 carbon atoms, for example zinc, magnesium or lithium stearate, zinc laurate or magnesium myristate, Polypore^{®} L200 (Chemdal Corporation), silicone resin microbeads (for example Tospearl^{®} from Toshiba) and polyurethane powders, in particular powders of crosslinked polyurethane comprising a copolymer, the said copolymer comprising trimethylol hexyllactone. It may in particular be a hexamethylene diisocyanate/trimethylol hexyllactone polymer. Such particles are especially commercially available, for example, under the name Plastic Powder D-400^{®} or Plastic Powder D-800^{®} from the company Toshiki, and mixtures thereof.

The filler(s) may be present in the composition of the invention in a content ranging from 0.01% to 10% by weight and in particular from 0.5% to 5% by weight relative to the total weight of the said composition.

### PHYSIOLOGICALLY ACCEPTABLE MEDIUM

Besides the compounds indicated previously, a composition according to the invention comprises a physiologically acceptable medium.

The term "physiologically acceptable medium" is intended to denote a medium that is particularly suitable for applying a composition of the invention to keratin materials, in particular the skin.

The physiologically acceptable medium is generally adapted to the nature of the support onto which the composition is to be applied, and also to the aspect in which the composition is to be conditioned.

A composition of the invention may be a dispersion or an emulsion.

A dispersion may be made as an aqueous phase or as an oily phase.

An emulsion may have an oily or aqueous continuous phase. Such an emulsion may be, for example, an inverse (W/O) emulsion or a direct (O/W) emulsion, or alternatively a multiple emulsion (W/O/W or O/W/O).

According to one preferred mode of the invention, the composition of the invention is in the form of a water-in-oil (W/O) emulsion.

### Aqueous phase

The composition according to the invention may comprise an aqueous phase.

The aqueous phase comprises water. A water that is suitable for use in the invention may be a floral water such as cornflower water and/or a mineral water such as Vittel water, Lucas water or La Roche Posay water and/or a spring water.

The aqueous phase may also comprise organic solvents that are water-miscible (at room temperature: 25°C), for instance monoalcohols containing from 2 to 6 carbon atoms, such as ethanol or isopropanol; polyols especially containing from 2 to 20 carbon atoms, preferably containing from 2 to 10 carbon atoms and preferentially containing from 2 to 6 carbon atoms, such as glycerol, propylene glycol, butylene glycol, pentylene glycol, hexylene glycol, dipropylene glycol or diethylene glycol; glycol ethers (especially containing from 3 to 16 carbon atoms) such as mono-, di- or tripropylene glycol (C1-C4)alkyl ethers, mono-, di- or triethylene glycol (C1-C4)alkyl ethers, and mixtures thereof.

The aqueous phase may also comprise stabilizers, for example sodium chloride, magnesium dichloride or magnesium sulfate.

The aqueous phase may also comprise any watersoluble or water-dispersible compound that is compatible with an aqueous phase, such as gelling agents, film-forming polymers, thickeners or surfactants, and mixtures thereof.

In particular, a composition of the invention may comprise an aqueous phase in a content ranging from 1% to 90% by weight, especially from 10% to 70% and more particularly from 20% to 60% by weight relative to the total weight of the composition.

According to another embodiment, a composition of the invention may be anhydrous.

An anhydrous composition may comprise less than 5% by weight of water relative to the total weight of the composition, in particular less than 3%, especially less than 2% and more particularly less than 1% by weight of water relative to the total weight of the composition. More particularly, an anhydrous composition may be free of water.

The vinyl polymer containing at least one carbosiloxane dendrimer-based unit is generally conveyed in the fatty phase of the composition of the invention.

### Fatty phase

A cosmetic composition in accordance with the present invention comprises at least one liquid fatty phase, especially at least one oil as mentioned below.

The term "oil" means any fatty substance that is in liquid form at room temperature (20-25°C) and at atmospheric pressure.

A composition of the invention may comprise a liquid fatty phase in a content ranging from 1% to 90%, in particular from 10% to 80%, in particular from 15% to 70% and more particularly from 20% to 50% by weight relative to the total weight of the composition.

The oily phase that is suitable for preparing the cosmetic compositions according to the invention may comprise hydrocarbon-based oils, silicone oils, fluoro oils or non-fluoro oils, or mixtures thereof.

The oils may be volatile or non-volatile.

They may be of animal, plant, mineral or synthetic origin.

For the purposes of the present invention, the term "volatile oil" means an oil (or non-aqueous medium) capable of evaporating on contact with the skin in less than one hour at room temperature and atmospheric pressure. The volatile oil is a volatile cosmetic oil, which is liquid at room temperature, especially having a non-zero vapour pressure, at room temperature and atmospheric pressure, in particular having a vapour pressure ranging from 0.13 Pa to 40 000 Pa (10⁻³ to 300 mmHg), preferably ranging from 1.3 Pa to 13 000 Pa (0.01 to 100 mmHg), and preferentially ranging from 1.3 Pa to 1300 Pa (0.01 to 10 mmHg).

For the purposes of the present invention, the term "non-volatile oil" means an oil with a vapour pressure of less than 0.13 Pa.

For the purposes of the present invention, the term "silicone oil" means an oil comprising at least one silicon atom, and especially at least one Si-O group.

The term "fluoro oil" means an oil comprising at least one fluorine atom.

The term "hydrocarbon-based oil" means an oil mainly containing hydrogen and carbon atoms.

The oils may optionally comprise oxygen, nitrogen, sulfur and/or phosphorus atoms, for example in the form of hydroxyl or acid radicals.

### Volatile oils

The volatile oils may be chosen from hydrocarbon-based oils containing from 8 to 16 carbon atoms, and especially C₈-C₁₆ branched alkanes (also known as isoparaffins), for instance isododecane (also known as 2,2,4,4,6-pentamethylheptane), isodecane and isohexadecane, for instance the oils sold under the trade names Isopar^{®} or Permethyl^{®}.

Volatile oils that may also be used include volatile silicones, for instance volatile linear or cyclic silicone oils, especially those with a viscosity ≤ 8 centistokes (cSt) (8 x 10⁻⁶ m /s), and especially containing from 2 to 10 silicon atoms and in particular from 2 to 7 silicon atoms, these silicones optionally comprising alkyl or alkoxy groups containing from 1 to 10 carbon atoms. As volatile silicone oils that may be used in the invention, mention may be made especially of dimethicones of viscosity 5 and 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Volatile fluoro oils such as nonafluoromethoxybutane or perfluoromethylcyclopentane, and mixtures thereof, may also be used.

According to one embodiment, a composition of the invention may comprise from 1% to 80% by weight, or even from 5% to 70% by weight, or even from 10% to 60% by weight and especially from 15% to 50% by weight of volatile oil relative to the total weight of the composition.

### Non-volatile oils

The non-volatile oils may be chosen especially from non-volatile hydrocarbon-based, fluoro and/or silicone oils.

Non-volatile hydrocarbon-based oils that may especially be mentioned include:
- hydrocarbon-based oils of animal origin, such as perhydrosqualene,
- hydrocarbon-based oils of plant origin, such as phytostearyl esters, such as phytostearyl oleate, phytostearyl isostearate and lauroyl/octyldodecyl/phytostearyl glutamate (Ajinomoto, Eldew PS203), triglycerides formed from fatty acid esters of glycerol, in particular in which the fatty acids may have chain lengths ranging from C₄ to C₃₆ and especially from C₁₈ to C₃₆, these oils possibly being linear or branched, and saturated or unsaturated; these oils may especially be heptanoic or octanoic triglycerides, shea oil, alfalfa oil, poppy oil, winter squash oil, millet oil, barley oil, quinoa oil, rye oil, candlenut oil, passionflower oil, shea butter, aloe vera oil, sweet almond oil, peach stone oil, groundnut oil, argan oil, avocado oil, baobab oil, borage oil, broccoli oil, calendula oil, camellina oil, canola oil, carrot oil, safflower oil, flax oil, rapeseed oil, cotton oil, coconut oil, marrow seed oil, wheatgerm oil, jojoba oil, lily oil, macadamia oil, corn oil, meadowfoam oil, St John's Wort oil, monoi oil, hazelnut oil, apricot kernel oil, walnut oil, olive oil, evening primrose oil, palm oil, blackcurrant pip oil, kiwi seed oil, grapeseed oil, pistachio oil, winter squash oil, pumpkin oil, quinoa oil, musk rose oil, sesame oil, soybean oil, sunflower oil, castor oil and watermelon oil, and mixtures thereof, or alternatively caprylic/capric acid triglycerides, such as those sold by the company Stearineries Dubois or those sold under the names Miglyol 810^{®}, 812^{®} and 818^{®} by the company Dynamit Nobel;
- linear or branched hydrocarbons of mineral or synthetic origin, such as liquid paraffins and derivatives thereof, petroleum jelly, polydecenes, polybutenes, hydrogenated polyisobutene such as Parleam, and squalane;
- synthetic ethers containing from 10 to 40 carbon atoms;
- synthetic esters, for instance the oils of formula R₁COOR₂, in which R₁ represents a linear or branched fatty acid residue containing from 1 to 40 carbon atoms and R² represents a hydrocarbon-based chain, which is especially branched, containing from 1 to 40 carbon atoms, on condition that R₁ + R₂ ≥ 10. The esters may be chosen especially from fatty acid esters of alcohols, for instance: cetostearyl octanoate, isopropyl alcohol esters, such as isopropyl myristate, isopropyl palmitate, ethyl palmitate, 2-ethylhexyl palmitate, isopropyl stearate, isopropyl isostearate, isostearyl isostearate, octyl stearate, hydroxylated esters, for instance isostearyl lactate, octyl hydroxystearate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate, 2-ethylhexyl palmitate, alkyl benzoates, polyethylene glycol diheptanoate, propylene glycol 2-diethylhexanoate, and mixtures thereof, C₁₂-C₁₅ alcohol benzoates, hexyl laurate, neopentanoic acid esters, for instance isodecyl neopentanoate, isotridecyl neopentanoate, isostearyl neopentanoate, octyldodecyl neopentanoate, isononanoic acid esters, for instance isononyl isononanoate, isotridecyl isononanoate, octyl isononanoate, hydroxylated esters, for instance isostearyl lactate and diisostearyl malate;
- polyol esters and pentaerythritol esters, for instance dipentaerythrityl tetra hydroxystea rate/tetraisostea rate,
- esters of diol dimers and of diacid dimers, such as Lusplan DD-DA5^{®} and Lusplan DD-DA7^{®} sold by the company Nippon Fine Chemical and described in patent application US 2004-175 338,
- copolymers of a diol dimer and of a diacid dimer and esters thereof, such as dilinoleyl diol dimer/dilinoleic dimer copolymers and esters thereof, for instance Plandool-G,
- copolymers of polyols and of diacid dimers, and esters thereof, such as Hailuscent ISDA or the dilinoleic acid/butanediol copolymer,
- fatty alcohols that are liquid at room temperature, with a branched and/or unsaturated carbonbased chain containing from 12 to 26 carbon atoms, for instance 2-octyldodecanol, isostearyl alcohol, oleyl alcohol, 2-hexyldecanol, 2-butyloctanol and 2-undecylpentadecanol,
- C₁₂-C₂₂ higher fatty acids, such as oleic acid, linoleic acid or linolenic acid, and mixtures thereof, and
- dialkyl carbonates, the two alkyl chains possibly being identical or different, such as dicaprylyl carbonate sold under the name Cetiol CC^{®} by Cognis,
- oils of high molar mass, in particular having a molar mass ranging from about 400 to about 10 000 g/mol, in particular from about 650 to about 10 000 g/mol, in particular from about 750 to about 7500 g/mol and more particularly ranging from about 1000 to about 5000 g/mol. As oils of high molar mass that may be used in the present invention, mention may be made especially of oils chosen from:
   ∘ lipophilic polymers,
   ∘ linear fatty acid esters with a total carbon number ranging from 35 to 70,
   ∘ hydroxylated esters,
   ∘ aromatic esters,
   ∘ C₂₄-C₂₈ esters of fatty alcohols or of branched fatty acids,
   ∘ silicone oils,
   ∘ oils of plant origin,
   ∘ and mixtures thereof;
- optionally partially hydrocarbon-based and/or silicone fluoro oils, for instance fluorosilicone oils, fluoropolyethers and fluorosilicones as described in document EP-A-847 752;
- silicone oils, for instance linear or cyclic non-volatile polydimethylsiloxanes (PDMS); polydimethylsiloxanes comprising alkyl, alkoxy or phenyl groups, which are pendant or at the end of a silicone chain, these groups containing from 2 to 24 carbon atoms; phenyl silicones, for instance phenyl tri-methicones, phenyl dimethicones, phenyl trimethylsiloxy diphenyl siloxanes, diphenyl dimethicones, diphenyl methyldiphenyl trisiloxanes and 2-phenylethyl trimethylsiloxy silicates, and
- mixtures thereof.

According to one embodiment, a composition of the invention may comprise from 0.5% to 50% by weight and preferably from 1% to 30% by weight of non-volatile oil relative to the total weight of the composition.

### Surfactants

The composition according to the invention, when it is in the form of an emulsion, generally comprises at least one surfactant, chosen especially from amphoteric, anionic, cationic and nonionic surfactants, used alone or as a mixture.

The surfactants are generally present in the composition in a proportion that may range, for example, from 0.3% to 15% by weight, in particular from 0.5% to 10% by weight and more particularly from 1% to 7% by weight of surfactants relative to the total weight of the composition.

Needless to say, the surfactant is chosen so as to effectively stabilize the emulsions more particularly under consideration according to the invention, namely of O/W, W/O or O/W/O type. This choice falls within the competence of a person skilled in the art.

Examples that may be mentioned for the O/W emulsions include nonionic surfactants, and especially esters of polyols and of fatty acids with a saturated or unsaturated chain containing, for example, from 8 to 24 carbon atoms and better still from 12 to 22 carbon atoms, and the oxyalkylenated derivatives thereof, i.e. derivatives containing oxyethylenated and/or oxypropylenated units, such as the glyceryl esters of C₈-C₂₄ fatty acids, and the oxyalkylenated derivatives thereof; the polyethylene glycol esters of C₈-C₂₄ fatty acids, and the oxyalkylenated derivatives thereof; the sorbitol esters of C₈-C₂₄ fatty acids, and the oxyalkylenated derivatives thereof; the sugar (sucrose, glucose or alkylglucose) esters of C₈-C₂₄ fatty acids, and the oxyalkylenated derivatives thereof; fatty alkyl ethers; the sugar ethers of C₈-C₂₄ fatty alcohols, and mixtures thereof.

Glyceryl esters of fatty acids that may especially be mentioned include glyceryl stearate (glyceryl mono-, di- and/or tristearate) (CTFA name: glyceryl stearate) or glyceryl ricinoleate, and mixtures thereof.

Polyethylene glycol esters of fatty acids that may especially be mentioned include polyethylene glycol stearate (polyethylene glycol mono-, di- and/or tristearate) and more especially polyethylene glycol 50 OE monostearate (CTFA name: PEG-50 stearate) and polyethylene glycol 100 OE monostearate (CTFA name: PEG-100 stearate), and mixtures thereof.

Mixtures of these surfactants may also be used, for instance the product containing glyceryl stearate and PEG-100 stearate, sold under the name Arlacel 165 by the company Uniqema, and the product containing glyceryl stearate (glyceryl mono-distearate) and potassium stearate, sold under the name Tegin by the company Goldschmidt (CTFA name: glyceryl stearate SE).

Fatty acid esters of glucose or of alkylglucose that may be mentioned in particular include glucose palmitate, alkylglucose sesquistearates, for instance methylglucose sesquistearate, alkylglucose palmitates, for instance methylglucose palmitate or ethylglucose palmitate, fatty esters of methylglucoside and more especially the diester of methylglucoside and of oleic acid (CTFA name: Methyl glucose dioleate); the mixed ester of methylglucoside and of the oleic acid/hydroxystearic acid mixture (CTFA name: Methyl glucose dioleate/hydroxysterate); the ester of methylglucoside and of isostearic acid (CTFA name: Methyl glucose isostearate); the ester of methylglucoside and of lauric acid (CTFA name: Methyl glucose laurate); the mixture of the monoester and diester of methylglucoside and of isostearic acid (CTFA name: Methyl glucose sesquiisostearate); the mixture of the monoester and diester of methylglucoside and of stearic acid (CTFA name: Methyl glucose sesquistearate) and in particular the product sold under the name Glucate SS by the company Amerchol, and mixtures thereof.

Examples of oxyethylenated ethers of a fatty acid and of glucose or of alkylglucose that may be mentioned include the oxyethylenated ethers of a fatty acid and of methylglucose, and in particular the polyethylene glycol ether of the diester of methyl glucose and of stearic acid containing about 20 mol of ethylene oxide (CTFA name: PEG-20 methyl glucose distearate), such as the product sold under the name Glucam E-20 distearate by the company Amerchol; the polyethylene glycol ether of the mixture of monoester and diester of methylglucose and of stearic acid containing about 20 mol of ethylene oxide (CTFA name: PEG-20 methyl glucose sesquistearate) and in particular the product sold under the name Glucamate SSE-20 by the company Amerchol, and the product sold under the name Grillocose PSE-20 by the company Goldschmidt, and mixtures thereof.

Examples of sucrose esters that may be mentioned include sucrose palmitostearate, sucrose stearate and sucrose monolaurate.

Examples of fatty alkyl ethers that may be mentioned include polyethylene glycol ethers of fatty alcohols containing from 8 to 30 carbon atoms and especially from 10 to 22 carbon atoms, such as polyethylene glycol ethers of cetyl alcohol, of stearyl alcohol or of cetearyl alcohol (mixture of cetyl alcohol and stearyl alcohol). Examples that may be mentioned include ethers comprising from 1 to 200 and preferably from 2 to 100 oxyethylene groups, such as those of CTFA name Ceteareth-20 and Ceteareth-30, and mixtures thereof.

Sugar ethers that may especially be mentioned are alkylpolyglucosides, for example decylglucoside, for instance the product sold under the name Mydol 10 by the company Kao Chemicals, the product sold under the name Plantaren 2000 by the company Henkel, and the product sold under the name Oramix NS 10 by the company SEPPIC; caprylyl/capryl glucoside, for instance the product sold under the name Oramix CG 110 by the company SEPPIC or under the name Lutensol GD 70 by the company BASF; laurylglucoside, for instance the products sold under the names Plantaren 1200 N and Plantacare 1200 by the company Henkel; cocoglucoside, for instance the product sold under the name Plantacare 818/UP by the company Henkel; cetostearyl glucoside optionally as a mixture with cetostearyl alcohol, sold, for example, under the name Montanov 68 by the company SEPPIC, under the name Tego-Care CG90 by the company Goldschmidt and under the name Emulgade KE3302 by the company Henkel; arachidyl glucoside, for example in the form of the mixture of arachidyl alcohol and behenyl alcohol and arachidyl glucoside, sold under the name Montanov 202 by the company SEPPIC; cocoylethyl-glucoside, for example in the form of the mixture (35/65) with cetyl alcohol and stearyl alcohol, sold under the name Montanov 82 by the company SEPPIC; and mixtures thereof.

For the W/O emulsions, hydrocarbon-based or silicone surfactants may be used.

According to one embodiment variant, silicone surfactants are preferred.

Examples of hydrocarbon-based surfactants that may be mentioned include polyester polyols, for instance PEG-30 dipolyhydroxystearate sold under the reference Arlacel P 135 by the company Uniqema, and polyglyceryl-2 dipolyhydroxystearate sold under the reference Dehymuls PGPH by the company Cognis.

Examples of silicone surfactants that may be mentioned include alkyldimethicone copolyols such as lauryl methicone copolyol sold under the name Dow Corning 5200 Formulation Aid by the company Dow Corning and cetyl dimethicone copolyol sold under the name Abil EM 90 by the company Goldschmidt, or the polyglyceryl-4 isostearate/cetyl dimethicone copolyol/hexyl laurate mixture sold under the name Abil WE 09 by the company Goldschmidt.

One or more co-emulsifiers may also be added thereto. The co-emulsifier may be chosen advantageously from the group comprising alkyl esters of polyols. Alkyl esters of polyols that may especially be mentioned include glycerol and/or sorbitan esters, for example the polyglyceryl-3 diisostearate sold under the name Lameform TGI by the company Cognis, polyglyceryl-4 isostearate, such as the product sold under the name Isolan GI 34 by the company Goldschmidt, sorbitan isostearate, such as the product sold under the name Arlacel 987 by the company ICI, sorbitan glyceryl isostearate, such as the product sold under the name Arlacel 986 by the company ICI, and mixtures thereof.

A crosslinked elastomeric solid organopolysiloxane comprising at least one oxyalkylene group, such as the product obtained according to the procedure of Examples 3, 4 and 8 of document US-A-5 412 004 and the examples of document US-A-5 811 487, especially the product of Example 3 (synthetic example) of patent US-A-5 412 004, and as sold under the reference KSG 21 by the company Shin-Etsu, may also be used as surfactants for W/O emulsions.

According to one particular mode, a composition according to the invention comprises at least one silicone surfactant, chosen in particular from dimethicone copolyols and alkyldimethicone copolyols, and mixtures thereof.

The silicone surfactant(s) are advantageously present in a content ranging from 0.5% to 5% by weight relative to the total weight of the said composition.

### ADDITIVES

A cosmetic composition according to the invention may also comprise any additive usually used in the field under consideration, chosen, for example, from gelling agents, gums, resins, dispersants, semicrystalline polymers, antioxidants, preserving agents, fragrances, neutralizers, antiseptics, UV-screening agents, cosmetic active agents, moisturizers and emollients, and mixtures thereof.

These additives may be present in the composition of the invention in a content ranging from 0.1% to 20% by weight and in particular from 1% to 10% by weight relative to the total weight of the said composition.

A composition according to the invention may especially be in the form of a composition for making up and/or caring for the skin or the lips, in particular a foundation.

According to one preferred mode, the composition according to the invention is in the form of a water-in-oil emulsion.

In particular, it is a makeup composition. Preferably, the composition according to the invention is a foundation.

The present invention is presented in greater detail in the examples described below, which are given merely as illustrations of the invention and should not be interpreted as limiting the invention.

### EXAMPLES

### Examples 1 to 4: Influence of the nature of the coating on the desired satin effect

Examples 1 to 4 of fluid foundations show that the fluoro coating, compared with other types of coating, can produce a satin makeup result, which also has, in combination with the vinyl polymer containing at least one carbosiloxane dendrimer-based unit, very good remanence properties.

| | | | | mass% |
|---|---|---|---|---|
| A1 | Dimethicone copolyol sold under the reference KF 6017 by the company Shin-Etsu | | | 2.00 |
| | Cetyl PEG/PPG-10/1 dimethicone sold under the reference Abil EM 90 by the company Goldschmidt | | | 1.00 |
| | Cyclohexasiloxane | | | **X** |
| | Isododecane | | | 4.00 |
| | Dicapryl ether sold under the reference Cetiol OE by the company Cognis | | | 2.00 |
| | Ethyl hexyl methoxycinnamate | | | 3.00 |
| A2 | Butyl acrylate copolymer containing dendritic silicone side chains: Tris((trimethylsiloxy)-siloxyethyldimethylsiloxy)silylpropyl methacrylate in isododecane (40/60) sold under the reference Dow Corning FA 4002 ID by Dow Corning | | | 10.00 |
| A3 | Cyclohexasiloxane | | | **Y** |
| | Coated yellow iron oxide * | | | 2.98 |
| | Coated red iron oxide * | | | 0.94 |
| | Coated black iron oxide * | | | 0.32 |
| | Coated titanium dioxide * | | | 6.76 |
| A4 | Nylon 12 powder sold under the reference Orgasol 2002 EXD NAT COS by the company Arkema | | | 2.00 |
| B | Demineralized water | | | 34.30 |
| | Butylene glycol | | | 6.00 |
| | Magnesium sulfate | | | 0.70 |
| C | Ethanol | | | 8.00 |
| | TOTAL | | | 100% |
| *: the nature of the coating is detailed in the table below. | | | | |

| | Example 1 (Invention) | Example 2 (comparative) | Example 3 (comparative) | Example 4 (comparative) |
|---|---|---|---|---|
| Nature of the coating | C9-15 FLUOROALCOHO L PHOSPHATE. Coated pigments from the company Daito Kasei Kogyo | DISODIUM STEAROYL GLUTAMATE +ALUMINIU M HYDROXIDE | ISOPROPYL TITANIUM TRIISO-STEARATE. Coated pigments | SILICA + DIMETHICON E+ ALUMINIUM HYDROXIDE (SA-LUCE). |
| | | | | mass% |
| | | Coated pigments from the company Miyoshi Kasei | from the company Kobo | Coated pigments from the company Miyoshi Kasei |
| % X cyclohexasiloxane | 8.50 | 8.50 | 6.50 | 6.50 |
| % Y cyclohexasiloxane | 7.50 | 7.50 | 9.50 | 9.50 |
| Total X+Y | 16.00 | 16.00 | 16.00 | 16.00 |

### Procedure

The constituents of phase A1 are weighed out in the main beaker and are stirred with a Moritz blender (1000 rpm) while maintaining at room temperature.

Next, phase A2 is added at room temperature, by stirring using a Moritz blender (1000 rpm) until homogenized.

Phase A3 is prepared separately by milling three times in a three-roll mill the mixture of pigments and of cyclohexasiloxane.

This phase A3 is then added, with continued stirring, along with phase A4.

The aqueous phase B is also prepared separately, by weighing out in a beaker the butylene glycol and the magnesium sulfate, and by adding water preheated to 95°C.

The aqueous phase is stirred using a magnetic bar until homogenized.

The emulsion is prepared at room temperature: the aqueous phase B is poured into the fatty phase while gradually increasing the stirring speed (Moritz blender) up to 4000 rpm. Stirring is continued for 10 minutes.

Phase C (ethanol) is finally added.

The product obtained is stirred using a Rayneri blender (paddles) for 10 minutes between 50 and 60 rpm.

### Measurement of the desired satin effect and of the remanence of the satin effect

### Measurement principle

The gloss of the skin is measured using the polarimetric camera, which is a black and white polarimetric imaging system, with which images are acquired in parallel (P) and crossed (C) polarized light. By analysing the image resulting from subtraction of the two images (P-C), the gloss is quantified, by measuring the average greyscale of the brightest 5% of pixels corresponding to the gloss areas.

### Test procedure

The test proceeds in the following manner:
16 women enter an air-conditioned waiting room (22°C ± 2°C) 15 minutes before the start of the test.

They are freed of makeup and an image of one of their cheeks is taken with the polarimetric camera. This image allows measurement of the gloss at T0 before applying makeup.

Next, 100 mg of foundation are weighed out on a watch glass, which is applied with the bare fingers to the half-face on which the measurement at T0 was taken. After a drying time of 15 minutes, an image of the made-up cheek is taken. This image allows measurement of the gloss just after applying makeup (Timm).

The models then return to the air-conditioned room for 3 hours.

Finally, an image of the made-up cheek after the waiting time of 3 hours is taken with the polarimetric camera. This image allows measurement of the gloss after 3 hours of makeup (T3h).

### Expressing the results

The difference (Timm - T0) which measures the effect of the makeup on the skin is calculated. A negative value means that the makeup reduces the gloss of the skin and that it thus has a matt effect.

A positive value means that the makeup increases the gloss of the skin. It thus leads to a satin effect, which is desired in the context of the invention. This satin effect is characterized by a gloss value, measured according to this protocol, of less than 5, preferably less than or equal to 4 and better still less than or equal to 3.

The difference (T3h - Timm) which measures the remanence of this effect is then calculated. The value obtained should be as low as possible, which means that the makeup effect does not change over time.

| | Example 1 (Invention) | Example 2 (comparative) | Example 3 (comparative) | Example 4 (comparative) |
|---|---|---|---|---|
| Nature of the coating | C9-15 FLUOROALCOHOL PHOSPHATE. Coated pigments from the company Daito Kasei Kogyo | DISODIUM STEAROYL GLUTAMATE +ALUMINIUM HYDROXIDE. Coated pigments from the company Miyoshi Kasei | ISOPROPYL TITANIUM TRIISO-STEARATE. Coated pigments from the company Kobo | SILICA + DIMETHICONE + ALUMINIUM HYDROXIDE (SA-LUCE). Coated pigments from the company Miyoshi Kasei |
| Effect of the makeup on the gloss of the skin (Timm-T0) | 2.69 | -2.62 | -4.12 | -5.56 |
| Remanence of the makeup effect (T3h-Timm) | 2.88 | 3.79 | 3.64 | 5.43 |

These results show that only the fluoro coating can produce a satin makeup result (positive value of the gloss measurement), the other coatings giving a matt makeup result (negative value of the gloss measurement). Good remanence of this satin effect is also obtained (low value close to that of the initial makeup result), by virtue of the combination with the final polymer containing at least one carbosiloxane dendrimer-based unit.

### Example 5: Influence of the nature of the Polymer on the remanence of the desired satin effect

| | | mass% |
|---|---|---|
| A1 | Dimethicone copolyol sold under the reference KF 6017 by the company Shin-Etsu | 2.00 |
| | Cetyl PEG/PPG-10/1 dimethicone sold under the reference Abil EM 90 by the company Goldschmidt | 1.00 |
| | Cyclohexasiloxane | 8.50 |
| | Isododecane | 4.00 |

| | | mass% |
|---|---|---|
| | Dicapryl ether sold under the reference Cetiol OE by the company Cognis | 2.00 |
| | Ethyl hexyl methoxycinnamate | 3.00 |
| A2 | **Polymer** | **10.00** |
| A3 | Cyclohexasiloxane | 7.50 |
| | Yellow iron oxide coated with C9-15 fluoroalcohol phosphate sold by the company Daito Kasei Kogyo under the reference PFX5 Sunpuro Yellow C33-9001 | 2.98 |
| | Red iron oxide coated with C9-15 fluoroalcohol phosphate sold by the company Daito Kasei Kogyo under the reference PFX5 Sunpuro Red C33-8001 | 0.94 |
| | Black iron oxide coated with C9-15 fluoroalcohol phosphate sold by the company Daito Kasei Kogyo under the reference PFX5 Sunpuro Black C33-7001 | 0.32 |
| | Titanium dioxide coated with C9-15 fluoroalcohol phosphate sold by the company Daito Kasei Kogyo under the reference PF5 TIO2 A 100 | 6.76 |
| A4 | Nylon 12 powder sold under the reference Orgasol 2002 EXD NAT COS by the company Arkema | 2.00 |
| B | Demineralized water | 34.30 |
| | Butylene glycol | 6.00 |
| | Magnesium sulfate | 0.70 |
| C | Ethanol | 8.00 |
| | TOTAL | 100% |

### Procedure

The compositions are prepared as described in the preceding examples.

| | Example 1 (Invention) | Example 5 (comparative) |
|---|---|---|
| Nature of the polymer | Butyl acrylate copolymer containing dendritic silicone side chains: Tris((trimethylsiloxy)siloxyethyldimethylsiloxy)silylpropyl methacrylate in isododecane (40/60) sold under the reference Dow Corning FA 4002 ID by Dow Corning. | Alkyl methacrylate copolymer containing silicone side chains in isododecane (40/60) sold under the reference KP 550 by the company Shin-Etsu. |
| Effect of the makeup on the gloss of the skin (Timm-T0) | 2.69 | 5.21 |
| Remanence of the makeup effect (T3h-Timm) | 2.88 | 4.11 |

These results show that the vinyl polymer containing at least one carbosiloxane dendrimer-based unit of the invention gives the best properties in terms of satin effect and remanence of the satin effect.

## Claims

1. Cosmetic composition for making up and/or caring for keratin materials, in particular the skin, comprising a liquid fatty phase, at least one vinyl polymer containing at least one carbosiloxane dendrimer-based unit and at least one particulate dyestuff coated with at least one fluoro compound ; the said vinyl polymer having as INCI name ACRYLATES/POLYTRIMETHYL SILOXYMETHACRYLATE COPOLYMER.

2. Cosmetic composition according to the preceding claim, **characterized in that** a vinyl polymer containing at least one carbosiloxane dendrimer-based unit is present in the composition in a content of greater than or equal to 1% by weight of active material relative to the total weight of the said composition.

3. Cosmetic composition according to either of the preceding claims, in which the said vinyl polymer containing at least one carbosiloxane dendrimer-based unit is the product of polymerization of :
(A) from 0 to 99.9 parts by weight of one or more acrylate or methacrylate monomers; and
(B) from 100 to 0.1 part by weight of an acrylate or methacrylate monomer of a tris[tri(trimethylsiloxy)-silylethyldimethylsiloxy]silylpropyl carbosiloxane dendrimer.

4. Cosmetic composition according to either of the preceding claims, in which the said vinyl polymer containing at least one carbosiloxane dendrimer-based unit comprises a tris[tri(trimethylsiloxy)silylethyldimethylsiloxy]sil-ylpropyl carbosiloxane dendrimer-based unit corresponding to one of the formulae: 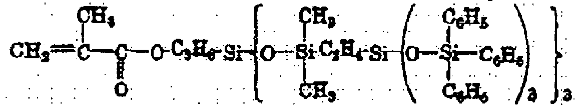 or

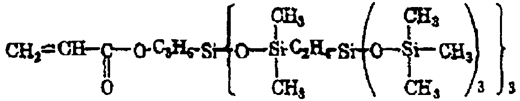

5. Composition according to any one of the preceding claims, in which the said vinyl polymer containing at least one carbosiloxane dendrimer-based unit comprises at least one butyl acrylate monomer.

6. Composition according to any one of the preceding claims, in which the said vinyl polymer containing at least one carbosiloxane dendrimer-based unit is present in a content ranging from 1% to 20% by weight relative to the total weight of the composition, in particular ranging from 2% to 15% by weight and more particularly ranging from 3% to 10% by weight of active material of vinyl polymer containing at least one carbosiloxane dendrimer-based unit relative to the total weight of the said composition.

7. Composition according to any one of the preceding claims, in which the particulate dyestuff coated with at least one fluoro compound is chosen from pigments and nacres, and mixtures thereof.

8. Composition according to the preceding claim, **characterized in that** the fluoro compound is chosen from perfluoroalkanes, perfluoroalkyl phosphates, perfluoroalkylsilanes, perfluoroalkylsilazanes, polyhexafluoropropylene oxides, perfluoropolyethers, polytetrafluoropolyethylene (PTFE), and polyorganosiloxanes comprising perfluoroalkyl groups, such as perfluoroalkyl dimethicones and perfluoroalkyl trialkoxysilanes, and mixtures thereof.

9. Composition according to either of Claims 7 and 8, in which the particulate dyestuff coated with at least one fluoro compound is chosen from iron oxides coated with perfluoroalkyl phosphate or with perfluoropolymethyl isopropyl ether and titanium dioxide coated with perfluoroalkyl phosphate, and mixtures thereof.

10. Composition according to any one of the preceding claims, in which the said particulate dyestuff coated with at least one fluoro compound is present in a content ranging from 0.5% to 40% by weight relative to the total weight of the said composition, preferably from 1% to 30% by weight relative to the total weight of the said composition and even more preferentially from 5% to 15% by weight relative to the total weight of the said composition.

11. Composition according to any one of the preceding claims, which is in the form of a water-in-oil emulsion.

12. Composition according to any one of the preceding claims, comprising at least one silicone surfactant chosen in particular from dimethicone copolyols and alkyldimethicone copolyols, and mixtures thereof.

13. Composition according to any one of the preceding claims, the said composition being a foundation.

14. Non-therapeutic process for making up and/or caring for keratin materials, in particular the skin, comprising at least the step of applying to the said keratin material at least one coat of a composition according to any one of Claims **1 to 13.**

## Patentansprüche

1. Kosmetische Zusammensetzung zum Make-up und/oder zur Pflege von Keratinmaterialien, insbesondere der Haut, umfassend eine flüssige Fettphase, mindestens ein Vinylpolymer, das mindestens eine auf Carbosiloxandendrimer basierende Einheit enthält, und mindestens einen teilchenförmigen Farbstoff, der mit mindestens einer Fiuorverbindung beschichtet ist ; das Vinylpolymer als INCI Name ACRYLATES/POLYTRIMETHYL SILOXYMETHACRYLATE COPOLYMER habend.

2. Kosmetische Zusammensetzung nach dem vorhergehenden Anspruch, **dadurch gekennzeichnet, dass** ein Vinylpolymer, das mindestens eine auf Carbosiloxandendrimer basierende Einheit enthält, in der Zusammensetzung in einem Gehalt größer gleich 1 Gew.-% Wirksubstanz, bezogen auf das Gesamtgewicht der Zusammensetzung, vorliegt.

3. Kosmetische Zusammensetzung nach einem der vorhergehenden Ansprüche, wobei das Vinylpolymer, bei der es sich um das Produkt der Polymerisation von:
(A) 0 bis 99,9 Gewichtsteilen eines oder mehreren Acrylate oder Methacrylate monomer und
(B) 100 bis 0,1 Gewichtsteilen eines Acrylate-Monomers oder Methacrylate-Monomers von eines Tris[tri(trimethyl-siloxy)silylethyldimethylsiloxy]silylpropyl-carbosiloxandendrimers.

4. Zusammensetzung nach einem der vorhergehenden Ansprüche, wobei das Vinylpolymer, das mindestens eine auf Carbosiloxandendrimer basierende Einheit enthält, mindestens eine auf Tris[tri(trimethylsiloxy)silylethyldimethylsiloxy]silylpropyl-carbosiloxandendrimer basierende Einheit umfasst, 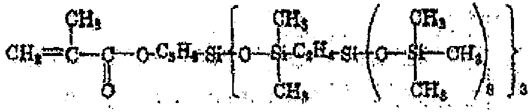 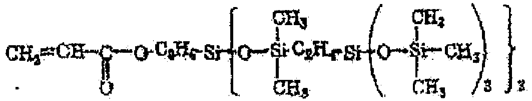 die einer der folgenden Formeln entspricht:

5. Zusammensetzung nach einem der vorhergehenden Ansprüche, wobei das Vinylpolymer, das mindestens eine auf Carbosiloxandendrimer basierende Einheit enthält, mindestens ein Butylacrylat-Monomer umfasst.

6. Zusammensetzung nach einem der vorhergehenden Ansprüche, wobei das Vinylpolymer, das mindestens eine auf Carbosiloxandendrimer basierende Einheit enthält, in einem Gehalt im Bereich von 1 bis 20 Gew.-%, bezogen auf das Gesamtgewicht der Zusammensetzung, insbesondere im Bereich von 2 bis 15 Gew.-% und spezieller im Bereich von 3 bis 10 Gew.-% Wirksubstanz von Vinylpolymer, das mindestens eine auf Carbosiloxandendrimer basierende Einheit enthält, bezogen auf das Gesamtgewicht der Zusammensetzung.

7. Zusammensetzung nach einem der vorhergehenden Ansprüche, wobei der mit mindestens einer Fluorverbindung beschichtete teilchenförmige Farbstoff aus Pigmenten und Perlmutten und Mischungen davon ausgewählt ist.

8. Zusammensetzung nach dem vorhergehenden Anspruch, **dadurch gekennzeichnet, dass** die Fluorverbindung aus Perfluor*alkanen, Perfluoralkylphosphaten, Perfluoralkylsilanen, Perfluoralkylsilazanen, Polyhexafluorpropylenoxiden, Perfluorpolyethern, Polytetrafluorpolyethylen (PTFE) und Polyorganosiloxanen mit Perfluoralkylgruppen, wie Perfluoralkyldimethiconen und Perfluoralkyltrialkoxysilanen, und Mischungen davon ausgewählt ist.

9. Zusammensetzung nach einem der Ansprüche 7 und 8, wobei der mit mindestens einer Fluorverbindung beschichtete teilchenförmige Farbstoff aus mit Perfluoralkylphosphat oder mit Perfluorpolymethylisopropylether beschichteten Eisenoxiden und mit Perfluoralkylphosphat beschichtetem Titandioxid und Mischungen davon ausgewählt ist.

10. Zusammensetzung nach einem der vorhergehenden Ansprüche, wobei der mit mindestens einer Fluorverbindung beschichtete teilchenförmige Farbstoff in einem Gehalt im Bereich von 0,5 bis 40 Gew.-%, bezogen auf das Gesamtgewicht der Zusammensetzung, vorzugsweise 1 bis 30 Gew.-%, bezogen auf das Gesamtgewicht der Zusammensetzung, und noch weiter bevorzugt 5 bis 15 Gew.-%, bezogen auf das Gesamtgewicht der Zusammensetzung, vorliegt.

11. Zusammensetzung nach einem der vorhergehenden Ansprüche, die in Form einer Wasser-in-Öl-Emulsion vorliegt.

12. Zusammensetzung nach einem der vorhergehenden Ansprüche, umfassend mindestens ein Silikon-Tensid, insbesondere ausgewählt aus Dimethiconcopolyolen und Alkyldimethiconcopolyolen, und Mischungen davon

13. Zusammensetzung nach einem der vorhergehenden Ansprüche, wobei es sich bei der Zusammensetzung um eine Grundierung handelt.

14. Nichttherapeutisches Verfahren zum Make-up und/oder zur Pflege von Keratinmaterialien, insbesondere der Haut, bei dem man auf das Keratinmaterial mindestens eine Schicht einer Zusammensetzung nach einem der Ansprüche **1 bis 13** aufbringt.

## Revendications

1. Composition cosmétique pour le maquillage et/ou le soin des matières kératiniques, en particulier la peau, comprenant une phase grasse liquide, au moins un polymère vinylique ayant au moins un motif dérivé de dendrimère carbosiloxane et au moins une matière colorante particulaire enrobée par au moins un composé fluoré ; ledit polymère vinylique ayant comme nom INCI ACRYLATES/POLYTRIMETHYL SILOXYMETHACRYLATE COPOLYMER.

2. Composition cosmétique selon la revendication précédente, **caractérisée en ce que** un polymère vinylique ayant au moins un motif dérivé de dendrimère carbosiloxane est présent dans la composition en une teneur supérieure ou égale à 1 % en poids de matière active par rapport au poids total de ladite composition.

3. Composition cosmétique selon l'une des revendications précédentes, dans laquelle ledit polymère vinylique est un produit de polymérisation de :
(A) de 0 à 99,9 parties en poids d'un ou plusieurs monomères acrylate ou methacrylate ; et
(B) de 100 to 0,1 parties en poids de monomère acrylate ou methacrylate d'un dendrimère carbosiloxane de tri[tri(trimethylsiloxy)-silylethyldimethylsiloxy]silylpropyle.

4. Composition cosmétique selon l'une des revendications précédentes, dans laquelle ledit polymère vinylique comprend un motif dérivé de dendrimère carbosiloxane tri[tri(trimethylsiloxy)silylethyldimethylsiloxy]sil-ylpropyle répondant à l'une des formules suivantes : 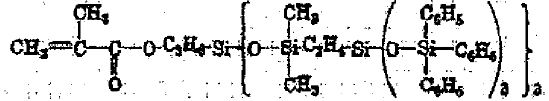 ou

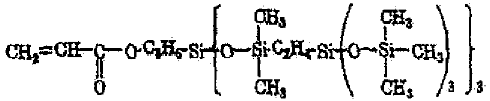

5. Composition selon l'une quelconque des revendications précédentes, dans laquelle ledit polymère vinylique ayant au moins un motif dérivé de dendrimère carbosiloxane comprend au moins un monomère acrylate de butyle.

6. Composition selon l'une quelconque des revendications précédentes, dans laquelle ledit polymère vinylique ayant au moins un motif dérivé de dendrimère carbosiloxane est présent en une teneur variant de 1 % à 20 % en poids par rapport au poids total de la composition, en particulier variant de 2 % à 15 % en poids, et plus particulièrement variant de 3 % à 10 % en poids en matière active de polymère vinylique ayant au moins un motif dérivé de dendrimère carbosiloxane par rapport au poids total de ladite composition.

7. Composition selon l'une quelconque des revendications précédentes, dans laquelle la matière colorante particulaire enrobée par au moins un composé fluoré est choisie parmi les pigments, les nacres et leurs mélanges.

8. Composition selon la revendication précédente, **caractérisée en ce que** le composé fluoré est choisi parmi les perfluoroalcanes, les perfluoroalkyl phosphates, les perfluoroalkyl silanes, les perfluoroalkyl silazanes, les polyoxydes d'hexafluoropropylène, les perfluoropolyéthers, le poly tétrafluoropolyéthylene (PTFE), les polyorganosiloxanes comprenant des groupes perfluoroalkyle comme les perfluoroalkyl diméthicones et les perfluoroalkyl trialcoxysilanes, et leurs mélanges.

9. Composition selon l'une des revendications **6 ou 7,** dans laquelle la matière colorante particulaire enrobée par au moins un composé fluoré est choisie parmi les oxydes de fer enrobés de phosphate de perfluoroalkyle ou de perfluoropolyméthylisopropyléther, le dioxyde de titane enrobé de phosphate de perfluoroalkyle, et leurs mélanges.

10. Composition selon l'une quelconque des revendications précédentes, dans laquelle ladite matière colorante particulaire enrobée par au moins un composé fluoré est présente en une teneur allant de 0,5 à 40 % en poids par rapport au poids total de ladite composition, de préférence de 1 à 30 % en poids par rapport au poids total de ladite composition, et encore plus préférentiellement de 5 à 15 % en poids par rapport au poids total de ladite composition.

11. Composition selon l'une quelconque des revendications précédentes, se présentant sous la forme d'une émulsion eau-dans-huile.

12. Composition selon l'une quelconque des revendications précédentes, comprenant au moins un agent tensioactif siliconé, en particulier choisi parmi les diméthicone copolyols, les alkyl-diméthicone copolyols et leurs mélanges.

13. Composition selon l'une quelconque des revendications précédentes, ladite composition étant un fond de teint.

14. Procédé non thérapeutique de maquillage et/ou de soin des matières kératiniques, en particulier la peau, comprenant au moins l'étape d'appliquer sur ladite matière kératinique, au moins une couche d'une composition selon l'une quelconque des revendications **1 à 13.**
